(19) Europäisches Patentamt
European Patent Office
Office européen des brevets

(11) Numéro de publication: **0 360 638 A1**

# (12) DEMANDE DE BREVET EUROPEEN

(21) Numéro de dépôt: **89402311.8**

(51) Int. Cl.[5]: **A 61 K 7/13**

(22) Date de dépôt: **21.08.89**

(30) Priorité: **08.09.88 LU 87333**

(43) Date de publication de la demande: **28.03.90 Bulletin 90/13**

(84) Etats contractants désignés: **AT BE CH DE ES FR GB GR IT LI NL SE**

(71) Demandeur: **L'OREAL**
**14, Rue Royale**
**F-75008 Paris (FR)**

(72) Inventeur: **Bore, Pierre**
**197 Avenue Daniel Perdrigé**
**F-93370 Montfermeil (FR)**

**De Labbey, Arnaud**
**9, rue Charles Dordain**
**F-93600 Aulnay-sous-Bois (FR)**

**Baudry, Alain**
**19 Villa du Buisson Ardent**
**F-95500 Gonesse (FR)**

(74) Mandataire: **Casalonga, Axel et al**
**BUREAU D.A. CASALONGA - JOSSE Morassistrasse 8**
**D-8000 München 5 (DE)**

## (54) Procédé de teinture des fibres kératiniques avec un dérivé d'indole associé à un nitrite et composition de mise en oeuvre.

(57) Procédé de teinture des fibres kératiniques, caractérisé par le fait que l'on applique sur ces fibres au moins un composition (A) contenant, dans un milieu approprié pour la teinture, au moins un dérivé d'indole répondant à la formule :

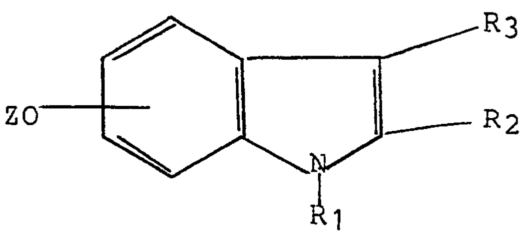

(I)

dans laquelle :
$R_1$ désigne un atome d'hydrogène, un radical alkyle en $C_1$-$C_4$;
$R_2$ ou $R_3$, identiques ou différents, désignent un atome d'hydrogène, un radical alkyle inférieur en $C_1$-$C_4$, un radical carboxyle, un radical alcoxycarbonyle;
ZO désigne un radical hydroxyle ou hydroxyle protégé, l'application de la composition (A) étant suivie par l'application d'une composition (B) constituée par une composition aqueuse présentant un pH acide, la composition (A) ou la composition (B) contenant au moins un nitrite.

EP 0 360 638 A1

## Description

### Procédé de teinture des fibres kératiniques avec un dérivé d'indole associé à un nitrite et composition de mise en oeuvre.

L'invention est relative à un nouveau procédé de teinture mettant en oeuvre les dérivés monohydroxylés d'indole associés à un nitrite et aux compositions mises en oeuvre dans ce procédé.

Les dérivés monohydroxylés d'indole sont bien connus dans l'état de la technique et ont déjà été préconisés pour la teinture des fibres kératiniques et en particulier des cheveux.

De telles utilisations sont décrites en particulier dans les brevets US-A-4.013.404, DE-A-3.031.709 et FR-A-2.252.841.

Ces procédés mettent généralement en oeuvre, lorsque les colorants sont utilisés comme colorants d'oxydation, du peroxyde d'hydrogène ou des persels, présentant l'inconvénient bien connu d'être dégradant pour le cheveu.

La demanderesse a découvert, de façon surprenante, qu'il était possible d'effectuer une teinture à l'aide de dérivés monohydroxylés d'indole, sans utiliser de peroxyde d'hydrogène ni de persel, en imprégnant, dans un premier temps, les fibres kératiniques, et en particulier les cheveux, par le dérivé d'indole monohydroxylé et, dans un deuxième temps, par une composition acide, l'une au moins des compositions contenant un nitrite.

Les teintures ainsi obtenues sont particulièrement intéressantes puisqu'elles permettent de teindre les cheveux dans les tons naturels, chauds, à reflets cuivrés, présentant une bonne résistance aux agents extérieurs, tels que la lumière, les intempéries et le lavage.

L'invention a donc pour objet un nouveau procédé de teinture des fibres kératiniques mettant en oeuvre des dérivés monohydroxylés d'indole et un nitrite.

Un autre objet de l'invention est constitué par les compositions et le dispositif à plusieurs compartiments utilisés dans le cadre de ce procédé.

D'autres objets de l'invention apparaîtront à la lecture de la description et des exemples qui suivent.

Le procédé de teinture des fibres kératiniques, en particulier des cheveux, conforme à l'invention, est essentiellement caractérisé par le fait que l'on applique sur ces fibres au moins une composition (A) contenant dans un milieu approprié pour la teinture, au moins un dérivé d'indole répondant à la formule :

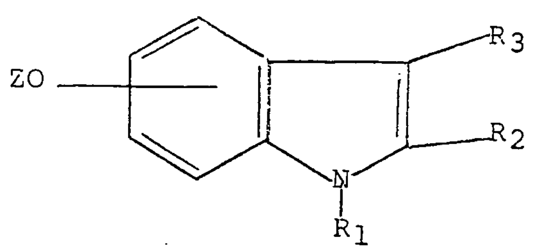

(I)

dans lequelle :

$R_1$ désigne un atome d'hydrogène ou un radical alkyle en $C_1$-$C_4$;

$R_2$ et $R_3$, identiques ou différents, désignent un atome d'hydrogène, un radical alkyle inférieur en $C_1$-$C_4$, un radical carboxyle, un radical alcoxycarbonyle;

ZO représente un groupement hydroxyle ou hydroxyle protégé;

l'application de la composition (A) étant suivie par l'application d'une composition aqueuse acide (B), la composition (A) ou la composition (B) contenant au moins un nitrite.

Dans la formule (I) précitée, Z désigne lorsque ZO représente un radical hydroxyle protégé, de préférence, un radical acyle en $C_2$-$C_6$.

Conformément à l'invention, les composés particulièrement préférés de formule (I), sont des composés dans lesquels le radical alkyle désigne méthyle, éthyle; le radical alcoxycarbonyle désigne méthoxy ou éthoxycarbonyle; le radical acyle désigne de préférence acétyle.

Parmi ces composés, on peut plus particulièrement citer le 6-hydroxyindole, le 6-hydroxy 3-carbométhoxyindole, le 6-hydroxy 1-méthyl 3-carbométhoxylindole, le 6-acétoxy 1-méthyl 3-carbométhoxy indole, le 6-acétoxy 1-méthyl 2,3-dicarbométhoxyindole, le 6-acétoxy, 1,2-diméthylindole, le 6-hydroxy 1,2-diméthyindole, le 6-hydroxy 2-méthylindole, le 6-hydroxy 2-carboxyindole, le 6-hydroxy 2,3-diméthyl indole, le 6-hydroxy 3-carboxyindole, le 6-hydroxy 3-carbéthoxyindole, le 6-hydroxy 2-carbéthoxyindole, le 6-acétoxyindole, le 6-hydroxy 3-méthylindole, le 5-hydroxyindole, le 7-hydroxyindole, le 4-hydroxyindole, le 6-hydroxy 1-méthylindole, le 5-hydroxy 2-carboxyindole.

Les nitrites tout particulièrement utilisables, conformément à l'invention, sont :

- des nitrites de métaux alcalins, alcalino-terreux ou d'ammonium et tout autre cation cosmétiquement acceptable lorsqu'il est utilisé pour le teinture des cheveux humains vivants;
- des dérivés organiques de nitrite, tels que par exemple le nitrite d'amyle; ou encore
- des vecteurs de nitrite, c'est-à-dire des composés qui, par transformation, génèrent un nitrite.

Les nitrites particulièrement préférés sont les nitrites de sodium, de potassium ou d'ammonium.

Les dérivés d'indole sont utilisés dans la composition (A) dans des quantités suffisantes pour teindre les

fibres kératiniques après association avec le nitrite, selon la nuance désirée. Cette concentration est comprise entre 0,01 et 0,3 mole/litre.

L'anion nitrite exprimé sous forme de $-NO_2^-$ est présent dans des quantités suffisantes pour développer avec les dérivés d'indole répondant à la formule (I), appliqués dans la première étape, une coloration. Sa concentration est de préférence comprise entre 0,02 et 1 mole/litre.

Le pH de la composition (A) est de préférence compris entre 2 et 10.

Le pH de la composition (B) est acide et permet de réguler la coloration.

En effet, en milieu acide, le nitrite est suffisamment oxydant pour réagir avec l'hydroxyindole ou son dérivé protégé.

Le pH de la composition (B) est compris de préférence entre 2 et 6 et en particulier il est ajusté à environ 3 pour des durées de contact faibles et pour des nuances les plus foncées.

Une forme de réalisation particulièrement préférée de l'invention consiste à appliquer, dans un premier temps, la composition (A) contenant le dérivé d'indole de formule (I) et, dans un second temps, la composition (B) contenant en milieu aqueux et acide, un nitrite.

Un autre forme de réalisation de l'invention peut consister à appliquer, dans un premier temps, une composition (A') contenant, dans un milieu approprié pour la teinture, le dérivé d'indole de formule (I) en milieu neutre ou alcalin et un nitrite. Dans ce cas, on teint les cheveux, en appliquant, dans un deuxième temps, une composition comprenant un milieu aqueux approprié pour la teinture ajusté à un pH acide et de préférence compris entre 2 et 6.

Un autre objet de l'invention est constitué par une telle composition (A') contenant simultanément les dérivés d'indole répondant à la formule (I), définis ci-dessus et les anions nitrite. Les proportions en dérivés d'indole de formule (I) et en nitrite, ainsi que la nature des nitrites, sont celles indiquées ci-dessus.

Les compositions (A) ou (B) ainsi que la composition (A') contenant les dérivés d'indole de formule (I) et le nitrite, peuvent contenir des adjuvants habituellement utilisés en teinture des fibres kératiniques et plus particulièrement en plus de l'eau, un solvant qui, dans le cas où la composition est appliquée sur les cheveux, est cosmétiquement acceptable.

Les solvants sont choisis parmi les solvants organiques, tels que les alcools inférieurs en $C_1$-$C_6$, l'alcool éthylique, l'alcool propylique ou isopropylique, l'alcool tertiobutylique, l'éthylène glycol, le propylèneglycol, les éthers monométhylique, monoéthylique ou monobutylique de l'éthylèneglycol, l'acétate du monoéthyléther de l'éthylèneglycol, les monométhyléthers du propylèneglycol et du dipropylène glycol, le lactate de méthyle.

Les solvants particulièrement préférés sont l'alcool éthylique et le propylèneglycol.

Les solvants sont utilisés plus particulièrement dans des concentrations comprises entre 10 et 50% pour les alcools inférieurs lorsque la concentration en dérivés d'indole de formule (I) dépasse 1% en poids par rapport au poids total de la composition.

Les compositions utilisables dans le procédé conforme à l'invention peuvent également contenir d'autres adjuvants qui sont cosmétiquement acceptables lorsque les compositions sont destinées à teindre les cheveux humains. Ces adjuvants sont, entre autres, des agents tensio-actifs anioniques, cationiques, non ioniques, amphotères ou leurs mélanges, présents de préférence dans des proportions comprises entre 0,1 et 50%; des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les épaississants peuvent être choisis parmi l'alginate de sodium, la gomme arabique, la gomme de guar, les biopolymères comme la gomme de xanthane, les scléroglucanes, les dérivés de cellulose tels que la méthylcellulose, l'hydroxyéthylcellulose, l'hydroxy propylméthylcellulose, le sel de sodium de la carboxyméthylcellulose et les polymères d'acide acrylique éventuellement réticulés.

On peut également utiliser des épaississants minéraux tels que la bentonite.

Ces épaississants utilisés seuls ou en mélange, sont présents de préférence dans des proportions comprises entre 0,1 et 5% en poids par rapport au poids total de la composition et avantageusement entre 0,5 et 3% en poids.

Les agents d'acidification utilisables sont plus particulièrement choisis parmi l'acide lactique, l'acide acétique, l'acide tartrique, l'acide phosphorique, l'acide chlorhydrique ou l'acide citrique.

Les agents alcalinisants sont particulièrement choisis parmi les amines, telles que les alcanolamines, les alkylamines ou alors les hydroxydes ou les carbonates alcalins ou d'ammonium.

Les compositions (A), (A') et/ou (B) utilisables dans le procédé conforme à l'invention, peuvent également contenir d'autres colorants habituellement utilisés pour la teinture des fibres kératiniques et en particulier le 5,6-dihydroxyindole ou ses dérivés, décrits plus particulièrement dans le brevet FR-A-2.595.245.

Les compositions peuvent également contenir des colorants directs, en particulier les dérivés nitrés benzéniques, des colorants d'oxydation, tels que des précurseurs de colorants d'oxydation du type para ou ortho, des coupleurs ou des colorants d'oxydation dits "rapides", c'est-à-dire des molécules à structure benzénique, précurseurs de colorants susceptibles de générer les composés colorés par simple oxydation à l'air pendant le temps de pose sur la chevelure, généralement inférieur à 1 heure et en absence d'un autre agent oxydant.

Les compositions peuvent être formulées sous des formes diverses cosmétiquement acceptables, lorsqu'elles sont appliquées sur les cheveux, telles que liquides plus ou moins épaissis ou gélifiés, crème, émulsion, mousse ou autres formes appropriées pour réaliser la teinture.

En vue de la mise en oeuvre du procédé conforme à l'invention, on peut conditionner les différentes compositions dans un dispositif à plusieurs compartiments, encore appelé "kit" ou nécessaire de teinture, comportant tous les composants destinés à être appliqués dans une même teinture sur les fibres kératiniques, et en particulier les cheveux, en applications successives avec ou sans prémélange.

De tels dispositifs sont connus en eux-mêmes et peuvent comporter un premier compartiment contenant la composition (A) renfermant le dérivé d'indole de formule (I) dans un milieu approprié pour la teinture, un deuxième compartiment, la composition (B) contenant dans un milieu approprié pour la teinture à un pH acide, un nitrite tel que défini ci-dessus.

Une autre forme de réalisation de l'invention consiste à prévoir un dispositif à plusieurs compartiments ou kits, comportant dans un premier compartiment, la composition (A') contenant dans un milieu approprié pour la teinture et présentant un pH neutre ou alcalin, le dérivé d'indole de formule (I) et les anions nitrite, et dans un second compartiment, une solution aqueuse présentant un pH acide compris de préférence entre 2 et 6.

Le procédé conforme à l'invention ainsi que les compositions définies ci-dessus, peuvent être mis en oeuvre pour teindre les cheveux naturels ou déjà teints, permanentés ou non, ou défrisés, ou des cheveux fortement ou légèrement décolorés, éventuellement permanentés.

Dans ce cas, on applique la composition (A) à une température qui est celle de la tête, c'est-à-dire comprise entre 25 et 35°C, pendant 5 à 30 minutes, et on fait suivre, avec ou sans rinçage intermédiaire, cette application par l'application de la composition (B) contenant le nitrite, qui est maintenue au contact des cheveux pendant 5 à 30 minutes. La température de la teinture est également celle de la tête et elle est comprise entre 25 et 35°C.

Dans la forme de réalisation mettant en oeuvre les dérivés d'indole de formule (I) et le nitrite dans une même composition en milieu neutre ou alcalin, on maintient la première composition (A') au contact des cheveux pendant 5 à 30 minutes et après rinçage éventuel, on applique la composition aqueuse acide.

Ces compositions peuvent également être utilisées pour la teinture des fourrures ou de la laine.

Les exemples suivants sont destinés à illustrer l'invention sans pour autant présenter un caractère limitatif.

## EXEMPLE 1

COMPOSITION (A) :

| | |
|---|---|
| - 4-hydroxyindole | 0,8 g |
| - Monobutyléther de l'éthylène glycol | 8,0 g |
| - Alkyléther de glycoside vendu à la concentration de 60% MA sous la dénomination de TRITON CG 110 per la Société SEPPIC | 2,4 g MA |
| - pH spontané = 4,7 | |
| - Eau qsp | 100,0 g |

COMPOSITION (B) :

| | |
|---|---|
| - Nitrite de sodium | 2,0 g |
| - Acide chlorhydrique qs pH = 3,8 | |
| - Eau qsp | 100,0 g |

La composition (A) est appliquée pendant 15 minutes sur des cheveux gris à 90% de blancs. Après rinçage, on applique pendant 10 minutes la composition (B). On rince et on sèche les cheveux. Ils sont alors colorés dans une nuance blond doré.

## EXEMPLE 2

COMPOSITION (A) :

| | |
|---|---|
| - 6-hydroxyindole | 1,5 g |
| - Ethanol | 12,0 g |
| - pH spontané = 6 | |
| - Eau qsp | 100,0 g |

COMPOSITION (B) :

| | |
|---|---|
| - Nitrite de sodium | 2,0 g |
| - Acide chlorhydrique qs pH = 3,8 | |
| - Eau qsp | 100,0 g |

La composition (A) est appliquée pendant 15 minutes sur des cheveux gris à 90% de blancs. Après rinçage, on applique pendant 10 minutes la composition (B) avant de rincer à nouveau. Après séchage, on obtient des cheveux teints dans une nuance blond acajou cuivré.

EXEMPLE 3

On prépare les compositions suivantes :

COMPOSITION (A) :

| | |
|---|---|
| - 4-hydroxyindole | 2,5 g |
| - Ethanol | 30,0 g |
| - Eau qsp | 100,0 g |
| - pH spontané = 6 | |

COMPOSITION (B) :

| | |
|---|---|
| - Nitrite de sodium | 2,5 g |
| - Acide chlorhydrique qs pH=3 | |
| - Eau qsp | 100,0 g |

La composition est appliquée pendant 15 minutes sur des cheveux gris à 90% de blancs. Après rinçage, on applique pendant 10 minutes la composition (B) avant de rincer à nouveau. Après séchage, on obtient des cheveux teints dans une nuance orangé foncé.

EXEMPLE 4

On prépare les compositions suivantes :

COMPOSITION (A) :

| | |
|---|---|
| - 5-hydroxyindole | 2,5 g |
| - Ethanol | 20,0 g |
| - Eau qsp | 100,0 g |
| - pH spontané = 5,75 | |

COMPOSITION (B) :

| | |
|---|---|
| - Nitrite de sodium | 2,5 g |
| - Acide chlorhydrique qs pH=3 | |
| - Eau qsp | 100,0 g |

On applique la composition (A) pendant 15 minutes sur des cheveux gris à 90% de blancs. Après rinçage, on applique pendant 9 minutes la composition (B) avant de rincer à nouveau. Après séchage, on obtient des cheveux teints dans une nuance violet lumineux très intense.

EXEMPLE 5

On prépare les compositions suivantes :

COMPOSITION (A) :

| | |
|---|---|
| - 5-hydroxy 2-carboxyindole | 2,5 g |
| - Ethanol | 40,0 g |
| - Eau qsp | 100,0 g |
| - pH spontané = 2,85 | |

COMPOSITION (B) :

| | |
|---|---|
| - Nitrite de sodium | 2,5 g |
| - Acide chlorhydrique qs pH=3 | |
| - Eau qsp | 100,0 g |

La composition (A) est appliquée pendant 15 minutes sur des cheveux gris à 90% de blancs. Après rinçage, on applique pendant 8 minutes la composition (B) avant de rincer à nouveau. Après séchage, on obtient des cheveux teints dans une nuance rouge vif.

EXEMPLE 6

On prépare les compositions suivantes :

COMPOSITION (A) :

| | |
|---|---|
| - 6-hydroxyindole | 2,5 g |
| - Ethanol | 10,0 g |
| - Eau qsp | 100,0 g |
| - pH spontané = 5,7 | |

COMPOSITION (B) :

| | |
|---|---|
| - Nitrite de sodium | 2,5 g |
| - Acide chlorhydrique qs pH=3 | |
| - Eau qsp | 100,0 g |

La composition (A) est appliquée pendant 15 minutes sur des cheveux gris à 90% de blancs. Après rinçage, on applique pendant 9 minutes la composition (B) avant de rincer à nouveau. Après séchage, on obtient des cheveux teints dans une nuance acajou cuivré.

EXEMPLE 7

On prépare les compositions suivantes :

COMPOSITION (A) :

| | |
|---|---|
| - 6-hydroxy 1-méthyl indole | 2,5 g |
| - Ethanol | 20,0 g |
| - Eau qsp | 100,0 g |
| - pH spontané = 5,8 | |

COMPOSITION (B) :

| | |
|---|---|
| - Nitrite de sodium | 2,5 g |
| - Acide chlorhydrique qs pH=3 | |
| - Eau qsp | 100,0 g |

On applique la composition (A) sur des cheveux gris à 90% de blancs pendant 15 minutes. Après rinçage, on applique pendant 10 minutes la composition (B) avant de rincer à nouveau. Après séchage, on obtient des cheveux teints dans une nuance acajou cuivré.

EXEMPLE 8

On prépare les compositions suivantes :

COMPOSITION (A) :

| | |
|---|---|
| - 7-hydroxyindole | 2,5 g |
| - Ethanol | 20,0 g |
| - Eau qsp | 100,0 g |
| - pH spontané = 5,75 | |

COMPOSITION (B) :

| | |
|---|---|
| - Nitrite de sodium | 2,5 g |
| - Acide chlorhydrique qs pH=3 | |
| - Eau qsp | 100,0 g |

On applique la composition (A) pendant 15 minutes sur des cheveux gris à 90% de blancs. Après rinçage, on applique pendant 9 minutes la composition (B) avant de rincer à nouveau. Après séchage, on obtient des cheveux teints dans une nuance jaune orangé.

EXEMPLE 9

On prépare les compositions suivantes :

COMPOSITION (A') :

| | |
|---|---|
| - 4-hydroxyindole | 2,5 g |
| - Nitrite de sodium | 2,5 g |
| - Dihydrogénophosphate de potassium | 1,3 g |
| - Monohydrogénophosphate de potassium | 3,6 g |
| - Ethanol | 30,0 g |
| - Eau qsp | 100,0 g |
| - pH spontané = 7,4 | |

COMPOSITION AQUEUSE ACIDE :

| | |
|---|---|
| - Citrate de sodium, dihydrate | 2,9 g |
| - Acide chlorhydrique qs pH=3 | |
| - Eau qsp | 100,0 g |

La composition (A') est appliquée pendant 30 minutes sur des cheveux à 90% de blancs. Après rinçage, on applique pendant 9 minutes la composition acide, avant de rincer à nouveau. Après séchage, on obtient des cheveux teints dans une nuance brun orangé.

**Revendications**

1. Procédé de teinture des fibres kératiniques, caractérisé par le fait que l'on applique sur ces fibres au moins une composition (A) contenant, dans un milieu approprié pour la teinture, au moins un dérivé d'indole répondant à la formule :

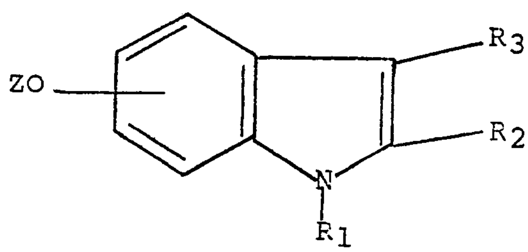

(I)

dans laquelle :
$R_1$ désigne un atome d'hydrogène, un radical alkyle en $C_1$-$C_4$;
$R_2$ ou $R_3$, identiques ou différents, désignent un atome d'hydrogène, un radical alkyle inférieur en $C_1$-$C_4$, un radical carboxyle, un radical alcoxycarbonyle;
ZO désigne un radical hydroxyle ou hydroxyle protégé,
l'application de la composition (A) étant suivie par l'application d'une composition (B) constituée par une composition aqueuse présentant un pH acide, la composition (A) ou la composition (B) contenant au moins un nitrite.

2. Procédé selon la revendication 1, caractérisé par le fait que dans la formule (I) des dérivés d'indole, Z désigne, lorsque ZO représente un groupement hydroxyle protégé, un radical acyle en $C_2$-$C_6$.

3. Procédé selon l'une quelconque des revendications 1 ou 2, caractérisé par le fait que les dérivés d'indole sont choisis parmi le 6-hydroxyindole, le 6-hydroxy 3-carbométhoxyindole, le 6-hydroxy 1-méthyl 3-carbométhoxyindole, le 6-acétoxy, 1-méthyl 3-carbométhoxyindole, le 6-acétoxy 1-méthyl 2,3-dicarbométhoxyindole, le 6-acétoxy, 1,2-diméthylindole, le 6-hydroxy 1,2-diméthylindole, le 6-hydroxy 2-méthylindole, le 6-hydroxy 2-carboxyindole, le 6-hydroxy 2,3-diméthyl indole, le 6-hydroxy 3-carboxyindole, le 6-hydroxy 3-carbéthoxyindole, le 6-hydroxy 2-carbéthoxyindole, le 6-acétoxyindole, le 6-hydroxy 3-méthylindole, le 5-hydroxyindole, le 7-hydroxyindole, le 4-hydroxyindole, le 6-hydroxy l-méthylindole, le 5-hydroxy 2-carboxyindole.

4. Procédé selon l'une quelconque des revendications 1 à 3, caractérisé par le fait que le nitrite est choisi parmi les nitrites des métaux alcalins, alcalino-terreux ou d'ammonium ou d'un autre cation cosmétiquement acceptable, ainsi que des dérivés organiques de nitrite ou des vecteurs de nitrite générant par transformation un nitrite.

5. Procédé selon l'une quelconque des revendications 1 à 4, caractérisé par le fait que le nitrite est

choisi parmi les nitrites de sodium, de potassium ou d'ammonium.

6. Procédé selon l'une quelconque des revendications 1 à 5, caractérisé par le fait que les dérivés d'indole de formule (I) sont utilisés dans des concentrations comprises entre 0,01 et 0,3 mole/litre.

7. Procédé selon l'une quelconque des revendications 1 à 6, caractérisé par le fait que le nitrite est utilisé dans des proportions comprises entre 0,02 et 1 mole/litre.

8. Procédé selon l'une quelconque des revendications 1 à 7, caractérisé par le fait que le pH de la composition (A) est compris entre 2 et 10.

9. Procédé selon l'une quelconque des revendications 1 à 8, caractérisé par le fait que le pH de la composition (B) est compris entre 2 et 6.

10. Procédé selon l'une quelconque des revendications 1 à 9, caractérisé par le fait que les compositions (A) ou (B) contiennent en plus de l'eau, au moins un ou plusieurs solvant(s) organique(s) cosmétiquement acceptable(s).

11. Procédé selon l'une quelconque des revendications 1 à 10, caractérisé par le fait que l'une au moins des compositions (A) ou (B) contient en plus le 5,6-dihydroxyindole ou un dérivé de celui-ci.

12. Procédé selon l'une quelconque des revendications 1 à 11, caractérisé par le fait que l'une au moins des compositions (A) ou (B) contient en plus un colorant direct, un colorant d'oxydation, un coupler ou un colorant d'oxydation rapide.

13. Procédé de teinture des fibres kératiniques selon l'une quelconque des revendications 1 à 12, caractérisé par le fait que l'on applique, dans un premier temps, une composition (A) contenant dans un milieu approprié pour la teinture, un dérivé d'indole répondant à la formule (I), et dans un second temps, une composition (B) contenant dans un milieu aqueux ajusté à un pH acide, des anions nitrite.

14. Procédé selon l'une quelconque des revendications 1 à 12, caractérisé par le fait que l'on applique, dans un premier temps, une composition (A') contenant dans un milieu approprié pour la teinture et présentant un pH neutre ou alcalin, un dérivé d'indole répondant à la formule (I) et des anions nitrite, et dans un second temps, on applique une composition aqueuse ajustée à un pH acide.

15. Procédé selon l'une quelconque des revendications 1 à 14, caractérisé par le fait que l'on tait suivre la première étape par une étape de rinçage.

16. Procédé selon l'une quelconque des revendications 1 à 15, caractérisé par le fait que la composition appliquée dans un premier temps est maintenue au contact des cheveux pendant une durée de 5 à 30 minutes et que la composition appliquée dans un second temps est maintenue au contact des cheveux pendant une durée également de 5 à 30 minutes.

17. Application du procédé selon l'une quelconque des revendications 1 à 16, à la teinture des cheveux humains.

18. Composition destinée à être utilisée pour la teinture des fibres kératiniques et en particulier des cheveux humains, caractérisée par le fait qu'elle contient dans un milieu aqueux à pH neutre ou alcalin, approprié pour la teinture, au moins un dérivé d'indole de formule (I), tel que défini dans l'une quelconque des revendications 1 à 3, et au moins des anions nitrite.

19. Dispositif à plusieurs compartiments ou "kit" destiné à être utilisé pour la teinture des fibres kératiniques et en particulier des cheveux humains, caractérisé par le fait qu'il comporte, dans un premier compartiment, une composition (A) contenant dans un milieu approprié pour la teinture, un dérivé d'indole de formule (I), tel que défini dans l'une quelconque des revendications 1 à 3, dans un compartiment (B), une composition aqueuse présentant un pH acide contenant un nitrite.

20. Dispositif à plusieurs compartiments ou "kit" destiné à être utilisé pour la teinture des fibres kératiniques et en particulier des cheveux humains, caractérisé par le fait qu'il comporte, dans un premier compartiment, une composition (A') telle que définie dans la revendication 18, et dans un second compartiment, une composition aqueuse acide.

Office européen des brevets

# RAPPORT DE RECHERCHE EUROPEENNE

Numero de la demande

EP 89 40 2311

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 271 186 (REPLIGEN CORP.)<br>* Revendications *<br>----- | 1,3,18 | A 61 K 7/13 |
| | | | **DOMAINES TECHNIQUES RECHERCHES (Int. Cl.5)** |
| | | | A 61 K |

Le présent rapport a été établi pour toutes les revendications

| Lieu de la recherche | Date d'achèvement de la recherche | Examinateur |
|---|---|---|
| LA HAYE | 23-11-1989 | WILLEKENS G.E.J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant